(19) Europäisches Patentamt
European Patent Office
Office européen des brevets

(11) Publication number: 0 319 962 A2

# (12) EUROPEAN PATENT APPLICATION

(21) Application number: 88120476.2

(22) Date of filing: 07.12.88

(51) Int. Cl.⁴: A61K 31/445

The title of the invention has been amended (Guidelines for Examination in the EPO, A-III, 7.3).

(30) Priority: 10.12.87 US 131160

(43) Date of publication of application: 14.06.89 Bulletin 89/24

(84) Designated Contracting States: AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Miller, Francis P.
336 Broadway
Loveland Ohio 45140(US)
Inventor: Carr, Albert A.
6693 East Farm Acres Drive
Cincinnati Ohio 45215(US)

(74) Representative: Macchetta, Francesco et al
Gruppo Lepetit S.p.A. Patent and Trademark Department 34, Via Roberto Lepetit
I-21040 Gerenzano (Varese)(IT)

## (54) Piperidinyl methanol derivatives for the treatment of anxiety.

(57) The present invention pertains to a method for the treatment of anxiety comprising administering a 1,4-disubstituted piperidinyl methanol to a patient in need thereof.

EP 0 319 962 A2

# METHOD FOR THE TREATMENT OF ANXIETY

The present invention pertains to a method for the treatment of anxiety.

In accordance with the present invention it has been discovered, that anxiety can be treated, that is relieved or alleviated, in a patient in need thereof, by the administration of an anxiolytic amount of a compound of the formula:

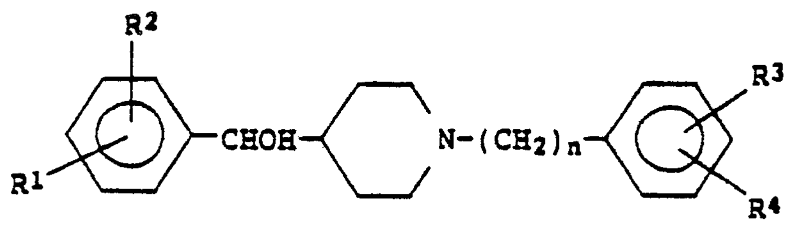

Formula I

wherein:

each of $R^1$, $R^2$, $R^3$, and $R^4$ is independently selected from the group consisting of hydrogen, a $C_{1-6}$ alkyl group, halogen, trifluoromethyl, hydroxy, a $C_{1-6}$ alkoxy group, or an amino group; n is 2, 3, or 4; and the pharmaceutically acceptable acid addition salts thereof.

As used in this application:

a) The term $C_{1-6}$ alkyl refers to a straight chain or branched alkyl group containing up to 6 carbon atoms. Representative examples of suitable alkyl groups include, methyl, ethyl, propyl, isopropyl, n-butyl, t-butyl, pentyl, hexyl, cyclopropyl, and cyclopentyl. Methyl and ethyl are currently preferred.

b) The term halogen refers to a fluorine, bromine, chlorine or iodine atom. Fluorine and chlorine are currently preferred.

c) The term $C_{1-6}$ alkoxy refers to a straight chain or branched alkoxy group containing up to 6 carbon atoms. Representative examples of suitable alkoxy groups include methoxy, ethoxy, propoxy, isopropoxy, butoxy, pentoxy, and hexyloxy.

d) The term hydroxy in this application refers to the following substituent -OH.

e) The term amino refers to $-NH_2$.

f) The term "patient" as used herein is taken to mean warm-blooded animals, such as mammals, for example, dogs, rats, mice, cats, guinea pigs, horses, cattle, sheep and primates, including humans.

g) The term anxiety refers to a condition where a patient is experiencing fear, apprehension, uncertainty, etc., and can be accompanied with physical manifestations such as, tachycardia, tremors, sweating, etc.

The expression "pharmaceutically acceptable acid addition salts" is intended to apply to any non-toxic organic or inorganic acid addition salt of the base compounds represented by Formula I. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicyclic, 2-phenoxybenzoic and sulfonic acids such as methane sulfonic acid and 2-hydroxyethane sulfonic acid.

Some of the compounds represented by Formula I exist as optical isomers. Any reference in this application to the compounds of Formula I, is meant to encompass a specific isomer or a mixture of isomers.

In those instances where $R^1$-$R^4$ are other than hydrogen, the substituents may be located at any position of the phenyl ring (i.e., meta, para, or ortho). Para is currently preferred for monosubstituted phenyl moieties. The 2,3-, 2,4- 2,5-, 3,4-, or 3,5-disubstituted phenyl moieties are also embraced herein.

$R^1$, $R^2$, $R^3$, and $R^4$ can each be the same or different substituents.

It is currently preferred for n to be either 2 or 3, with 2 being most preferred. It is also currently preferred for $R^3$ and $R^4$ to be hydrogen.

Representative examples of preferred compounds include:

1) α-phenyl-1-(2-phenethyl)-4-piperidine methanol;
2) α-phenyl-1-(3-phenylpropyl)-4-piperidine methanol;
3) α-(4-methylphenyl)-1-(2-phenethyl)-4-piperidine methanol;
4) α-(4-methoxyphenyl)-1-(2-phenethyl)-4-piperidine methanol;
5) α-(3,5-dimethylphenyl)-1-(2-phenethyl)-4-piperidine methanol;
6) α-(3-(trifluoromethyl)phenyl)-1-(2-phenethyl)-4-piperidine methanol;
7) α-(2,3-dimethoxyphenyl)-1-(2-phenethyl)-4-piperidine methanol;
8) α-(4-fluorophenyl)-[1-(2-phenylethyl)-4-piperidinyl]-methanol;
9) α-phenyl-[1-(4-phenylbutyl)-4-piperidinyl]-methanol;
10) α-(3,4-dimethoxyphenyl-[1-(2-phenylethyl)-4-piperidinyl]-methanol;
11) α-phenyl-[1-(4-aminophenylethyl)-4-piperidinyl]-methanol;
12) α-phenyl[l-(4-methoxyphenylethyl)-4-piperidinyl]-methanol;
13) α-(4-methoxyphenyl)-[1-(4-methoxyphenylethyl)-4-piperidinyl]-methanol;
14) α-(2,3-dimethoxyphenyl)-[1-(4-methoxyphenylethyl)-4-piperidinyl]-methanol;
15) α-phenyl-[1-(4-methoxyphenylethyl)-4-piperidinyl]-methanol;
16) α-phenyl-[1-(4-fluorophenylethyl)-4-piperidinyl]-methanol;
17) α-(4-hydroxyphenyl)-[1-(2-phenylethyl)-4-piperidinyl]-methanol;
18) α-(3,4-dihydroxyphenyl)-[1-(2-phenylethyl)-4-piperidinyl]-methanol;
19) α-(3,4-dichlorophenyl)-1-(2-phenylethyl)-4-piperidine methanol.

The compounds of Formula I, their methods of preparation and their use as serotonin $5HT_2$ antagonists are known in the art. European Patent Application 0 208 235 discloses these compounds and several methods for preparing these compounds. Any of these methods, or any other method known in the art, are suitable for preparing the compounds to be utilized in the method of the present invention.

The compounds of Formula I are useful in the treatment of anxiety; that is relieving or alleviating the apprehension, fear, or uncertainty, etc., that patients suffering from anxiety commonly experience, as well as relieving or alleviating the physiological changes associated with anxiety such as tachycardia, tremors, sweating, etc.

The compounds of Formula I possess a significant advantage over the anxiolytic agents which are currently available to clinicians, such as chlordiazepoxide, diazepam, and other benzodiazepines. The benzodiazepines commonly cause sedation and impairment of motor skills at the dosage levels commonly used in the treatment of anxiety.

The compounds of Formula I do not suffer from this disadvantage. They exhibit a wide dosage range at which they demonstrate anxiolytic activity, without causing either sedation or impairment of motor skills.

However, at elevated dosage ranges the compounds can cause sedation and impairment of motor skills. These elevated dosage ranges are not required to achieve a therapeutically effective anxiolytic effect, and therefore patients being treated with these compounds should not experience these side effects.

The quantity of compound required to produce the anxiolytic effect described above will vary with the particular compound utilized, the patient, the route of administration, the severity of the patient's anxiety, the presence of other underlying disease states in the patient, and other medications which are being administered concurrently to the patient. Generally though, a patients anxiety will respond to dosage range of from 0.25 to 25 mg/kg/day.

The compounds of Formula I can be administered either orally or parenterally. Repetitive daily administration may be desirable and will vary with the dosage form utilized as well as the other parameters described above for the quantity of compound required.

The compounds of Formula I can be compounded into a variety of dosage forms, such as for example, tablets, capsules, solutions, elixirs, sterile solutions for injection and sustained release preparations. Methods for producing these dosage forms are well known in the art and are disclosed in European Patent Application 0208235.

## Claims

1. A compound of the formula

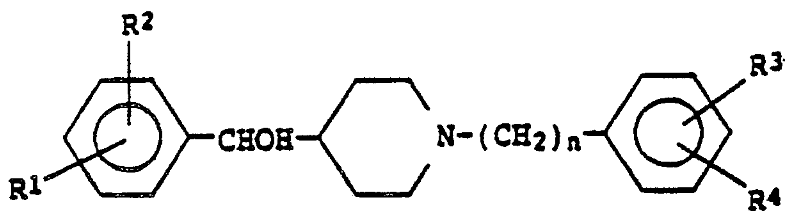

**Formula I**

wherein:
each of $R^1$, $R^2$, $R^3$, and $R^4$ is independently selected from the group consisting of hydrogen, a $C_{1-6}$ alkyl group, halogen, trifluoromethyl, hydroxy, a $C_{1-6}$ alkoxy group, or an amino group; n is 2, 3, or 4; and the pharmaceutically acceptable acid addition salts thereof, for use in the preparation of a medicament for the treatment of anxiety.

2. The method of claim 1, wherein said compound is α-phenyl-1-(2-phenethyl)-4-piperidine methanol.

3. The method of claim 1, wherein said compound is α-phenyl-1-(3-phenpropyl)-4-piperidine methanol.

4. The method of claim 1, wherein said compound is α-(4-methylphenyl)-1-(2-phenethyl)-4-piperidine methanol.

5. The method of claim 1, wherein said compound is α-(4-methoxyphenyl)-1-(2-phenethyl)-4-piperidine methanol.

6. The method of claim 1, wherein said compound is α-(3,5-dimethylphenyl)-1-(2-phenethyl)-4-piperidine methanol.

7. The method of claim 1, wherein said compound is α-(3-(trifluoromethyl)phenyl)-1-(2-phenethyl)-4-piperidine methanol.

8. The method of claim 1, wherein said compound is α-(2,3-dimethoxylphenyl)-1-(2-phenethyl)-4-piperidine methanol.

9. The method of claim 1, wherein said compound is α-(4-fluorophenyl)-[1-(2-phenylethyl)-4-piperidinyl]-methanol.

10. The method of claim 1, wherein said compound is α-phenyl-[1-(4-phenylbutyl)-4-piperidinyl]-methanol.

11. The method of claim 1, wherein said compound is α-(3,4-dimethoxyphenyl)-[1-(2-phenylethyl)-4-piperidinyl]-methanol.

12. The method of claim 1, wherein said compound is α-phenyl-[1-(4-aminophenylethyl)-4-piperidinyl]-methanol.

13. The method of claim 1, wherein said compound is α-phenyl[1-(4-methoxyphenylethyl)-4-piperidinyl]-methanol.

14. The method of claim 1, wherein said compound is α-(4-methoxyphenyl)-[1-(4-methoxyphenylethyl)-4-piperidinyl]-methanol.

15. The method of claim 1, wherein said compound is α-(2,3-dimethoxyphenyl)-[1-(4-methoxyphenylethyl)-4-piperidinyl]-methanol.

16. The method of claim 1, wherein said compound is α-phenyl-[1-(4-methoxyphenylethyl)-4-piperidinyl]-methanol.

17. The method of claim 1, wherein said compound is α-phenyl-[1-(4-fluorophenylethyl)-4-piperidinyl]-methanol

18. The method of claim 1, wherein said compound is α-(4-hydroxyphenyl)-[1-(2-phenylethyl)-4-piperidinyl]-methanol.

19. The method of claim 1, wherein said compound is α-(3,4-dihydroxyphenyl)-[1-(2-phenylethyl)-4-piperidinyl]-methanol.

20. The method of claim 1 wherein said compound is α-(3,4-dichlorophenyl)-1-(2-phenylethyl)-4-piperidine methanol.